# EUROPÄISCHE PATENTSCHRIFT

(11) **EP 1 682 580 B1**
(45) Veröffentlichungstag und Bekanntmachung des Hinweises auf die Patenterteilung: **15.05.2013**
(21) Anmeldenummer: 04802719.7
(22) Anmeldetag: 12.11.2004
(51) Int. Cl.: C07K 16/44

(54) **HUMANER MONOKLONALER ANTIKÖRPER MIT FETTSENKENDER WIRKUNG**
HUMAN MONOCLONAL ANTIBODY HAVING FAT-REDUCING EFFECT
ANTICORPS MONOCLONAL HUMAIN REDUCTEUR DE GRAISSE

(30) Priorität: 14.11.2003 DE 10353175
(43) Veröffentlichungstag der Anmeldung: 26.07.2006
(60) Teilanmeldung: 10194202.7
(73) Patentinhaber: PATRYS LIMITED, Melbourne, VIC 3000 (AU)
(72) Erfinder: VOLLMERS, H. Peter, 97078 Würzburg (DE)
(74) Vertreter: Minderop, Ralph H.
(86) Internationale Anmeldenummer: PCT/DE2004/002503
(87) Internationale Veröffentlichungsnummer: WO 2005/049635

(56) Entgegenhaltungen:
- EP-A- 0 592 106
- WO-A-03/048321
- GETZ G S: "The first human monoclonal antibody to oxidized LDL." ARTERIOSCLEROSIS, THROMBOSIS, AND VASCULAR BIOLOGY. AUG 2001, Bd. 21, Nr. 8, August 2001 (2001-08), Seiten 1254-1255, XP002333218 ISSN: 1524-4636 -& SHAW PETER X ET AL: "Human-derived anti-oxidized LDL autoantibody blocks uptake of oxidized LDL by macrophages and localizes to atherosclerotic lesions in vivo" ARTERIOSCLEROSIS THROMBOSIS AND VASCULAR BIOLOGY, Bd. 21, Nr. 8, August 2001 (2001-08), Seiten 1333-1339, XP002333371 ISSN: 1079-5642
- POHLE T ET AL: "Lipoptosis: Tumor-specific cell death by antibody-induced intracellular lipid accumulation" CANCER RESEARCH, AMERICAN ASSOCIATION FOR CANCER RESEARCH, BALTIMORE, MD, US, Bd. 64, Nr. 11, 1. Juni 2004 (2004-06-01), Seiten 3900-3906, XP002324184 ISSN: 0008-5472
- VOLLMERS H PETER ET AL: "Lipoptosis, tumor-specific cell death via lipid accumulation induced by human monoclonal IgM antibody SAM-6" HUMAN ANTIBODIES, Bd. 13, Nr. 1-2, 2004, Seite 3, XP009049406 & 11TH INTERNATIONAL CONFERENCE ON HUMAN ANTIBODIES AND HYBRIDOMAS; DUBLIN, IRELAND; OCTOBER 06-08, 2004 ISSN: 1093-2607
- BEAUMONT J L ET AL: "IMMUNOGLOBULIN-BOUND LIPOPROTEINS IG-LP AS MARKERS OF FAMILIAL HYPERCHOLESTEROLEMIA XANTHOMATOSIS AND ATHEROSCLEROSIS", ATHEROSCLEROSIS, vol. 74, no. 3, 1988, pages 191-202, ISSN: 0021-9150

### Sequence listing

<110> Vollmers, Philip
<120> Human monoclonal antibody
<150> DE-10 353 175.0
   <151> 2003-11-14
<160> 4
<210> 1
   <211> 96
   <212> DNA
   <213> Homo sapiens
<220>
   <223> Amino acid sequence of the variable region of the light chain (V_{L}) of antibody SAM-6
<400> 1
   Ser Tyr Val Leu Thr Gln Pro Pro Ser Val Ser Val Ser Pro Gly
   1               5                   10                  15
   Gln Thr Ala Ser Ile Thr Cys Ser Gly Asp Lys Leu Gly Asp Lys
                   20                  25                  30
   Tyr Ala Cys Trp Tyr Gln Gln Lys Pro Gly Gln Ser Pro Val Leu
                   35                  40                  45
   Val Ile Tyr Gln Asp Ser Lys Arg Pro Ser Gly Ile Pro Glu Arg
                   50                  55                  60
   Phe Ser Gly Ser Asn Ser Gly Asn Thr Ala Thr Leu Thr Ile Ser
                   65                  70                  75
   Gly Thr Gln Ala Met Asp Glu Ala Asp Tyr Tyr Cys Gln Ala Trp
                   80                  85                  90
   Asp Ser Ser Ile Val Val
                   95
<210> 2
   <211> 288
   <212> DNA
   <213> Homo sapiens
<220>
   <223> Nucleotide sequence ofthe variable region of the light chain (V_{L}) of antibody SAM-6
<400> 2
<210> 3
   <211> 110
   <212> DNA
   <213> Homo sapiens
<220>
   <223> Amino acid sequence of the variable region of the heavy chain (V_{H}) of antibody SAM-6
<400> 3
   Gln Val Gln Leu Val Glu Ser Gly Gly Gly Val Val Gln Pro Gly
   1               5                   10                  15
   Arg Ser Leu Arg Leu Ser Cys Ala Ala Ser Gly Phe Thr Phe Ser
                   20                  25                  30
   Ser Tyr Ala Met His Trp Val Arg Glu Ala Pro Gly Lys Gly Leu
                   35                  40                  45
   Glu Trp Val Ala Val Ile Ser Tyr Asp Gly Ser Asn Lys Tyr Tyr
                   50                  55                  60
   Ala Asp Ser Val Lys Gly Arg Phe Thr Ile Ser Arg Asp Asn Ser
                   65                  70                  75
   Lys Asn Thr Leu Tyr Leu Gln Met Asn Ser Leu Arg Ala Glu Asp
                   80                  85                  90
   Thr Ala Val Tyr Tyr Cys Ala Arg Asp Arg Leu Ala Val Ala Gly
                   95                  100                 105
   Lys Thr Phe Asp Tyr
                   110
<210> 4
   <211> 330
   <212> DNA
   <213> Homo sapiens
<220>
   <223> Nucleotide sequence of the variable region of the heavy chain (V_{H}) of antibody SAM-6
<400> 4

## Beschreibung

Die Erfindung betrifft ein aufgereinigtes Polypeptid (SAM-6.10) sowie dessen Verwendung in Kombination mit üblichen Hilfs- und/oder Trägerstoffen zur Herstellung eines Arzneimittels mit fettsenkender Wirkung sowie zur Herstellung eines Arzneimittels zur Behandlung von Nierenerkrankungen.

### Hintergrund der Erfindung

Bei einem an Überangebot an Cholesterin (Hyperlipoproteinämie) im Körper kommt es zur Verkalkung (arteriosklerotische Plaque) der Innenschichten der Gefäße und zu einer langsam fortschreitenden Verhärtung und Verdickung der Arterienwände. Im Extremfall droht ein Verschluss des Gefäßes oder beim Aufbrechen der Plaque Thrombusbildung. Die Arteriosklerose ist mit ihren Folgeerkrankungen (koronare Herzkrankheiten, Herzinfarkt, periphere arterielle Verschlusskrankheiten, Schlaganfall) noch immer die häufigste Todesursache in der westlichen Welt. Weit über die Hälfte aller für die medizinische Betreuung zur Verfügung stehenden fianziellen Mittel werden schätzungsweise für die Folgen der Arteriosklerose ausgegeben. Um die Ursachen der Arteriosklerose zu klären, wurden verschiedene Theorien entwickelt, wobei die Lipidtheorie die meistbeachtetste ist.

Allgemein läßt sich sagen: Je höher der LDL-Cholesteringehalt bzw. der Gehalt an oxidiertem LDL-Cholesterin im Blut, desto höher das Risiko, an einer Gefäßverkalkung beispielsweise mit der Folge eines Herzinfarktes zu erkranken. Übergewicht und Hypercholesterinämie sind mit die wichtigsten Risikofaktoren für die Entwicklung einer Arteriosklerose.

### Definitionen und Begriffe

Fette, wie z. B. Cholesterin, sind weder in Wasser noch in Blutflüssigkeit löslich. Um sie trotzdem in einzelne Körperregionen transportieren zu können, werden die Fette, sobald sie sich im Blut befinden, an bestimmte Eiweißkörper (Proteine) gebunden. Diese Verbindungen aus Lipiden (Fetten) und Proteinen (Eiweißen) werden als Lipoproteine bezeichnet.

Die "Lipoproteine" des Plasmas sind hochmolekulare wasserlösliche Komplexe, die aus Lipiden (Cholesterin, Triglyceride, Phospolipide) und Apolipoproteinen bestehen. Das cholesterinhaltige Lipoprotein LDL-Cholesterin verursacht Arteriosklerose und wird auch das "böse" Cholesterin genannt,

"Cholesterin" wird im Körper ubiquitär synthetisiert und ist ein wesentlicher Bestandteil von Zellmembranen und Lipoproteinen. Im Gegensatz zu den ebenfalls endogen synthetisierten Triglyceriden und Phospholipiden kann der Sterolring des Cholesterinmoleküls nicht mehr abgebaut werden; Cholesterin wird in der Leber zu Gallensäure umgewandelt oder unverändert über die Galle in den Darm ausgeschieden.

Im Plasma liegt Cholesterin zu 25-40% als freies (unverestertes) Cholesterin und zu 60-75% mit ungesättigten Fettsäuren verestert vor. Beide Formen zusammen werden als Gesamtcholesterin bezeichnet. Wegen seiner geringen Wasserlöslichkeit wird Cholesterin im Plasma als Komplex mit Apolipoproteinen transportiert. Im Blut werden etwa 70 Prozent des Gesamt-Cholesterins mit Hilfe von Low-Density-Lipoproteinen (LDL) transportiert.

"Triglyceride" sind Ester von Glycerin mit drei Fettsäureresten. Analog zum Cholesterin werden auch die Triglyceride im Plasma aufgrund ihrer schweren Löslichkeit an Apolipoproteine gebunden transportiert.

"Lipoproteine" werden in der Leber oder im Darm synthetisiert und transportieren im Blut fettlösliche Substanzen wie das Cholesterin.

Die Einteilung der Lipoproteine erfolgt nach ihrer Dichte, man unterscheidet die fünf Dichteklassen: Chylomikronen, very low density lipoproteines (VDL), low density lipoproteines (LDL) und high density lipoproteines (HDL). Die Chylomikronen, deren physiologische Konzentration im Nüchternserum im Gegensatz zu jenen der anderen Lipoproteine sehr gering ist, sind Transportvehikel für exogene Glyceride. Die physiologische Verteilung der anderen Lipoproteine ist wie folgt: VLDL 10 %, LDL 70 % und HDL 20 %. VLDL sind die Vorläufer der LDL und Vehikel für den Transport von endogenen Glyceriden. LDL entstehen durch die Hydrolyse der VLDL. LDL und HDL sind beides Regulatoren der zellulären Cholesterinhomöostase, wobei HDL außerdem die Lipolyse (Spaltung von Triglyzeriden in Glyzerin und die freien Fettsäuren) regulieren. LDL haben einen Durchmesser von ca. 20 nm. HDL sind die kleinsten (7-10 nm) und eiweißreichsten Lipoproteinen. Neben nativem LDL (LDL) ist im Blutserum auch oxidiertes LDL (oxLDL) nachweisbar. oxLDL interagiert mit endogenen Plasmaproteinen, insbesondere Glycoproteinen, durch spezifische Liganden und bildet oxLDL-Glykoprotein-Komplexe.

"Apolipoproteine" sind ein Bestandteil der Lipoproteine und umgeben, zusammen mit polaren Lipiden, als eine Art äußere Schale den aus hydrophoben Lipiden aufgebauten Lipoprotein-Kern. Mit Ausnahme von LDL, welches nur Apoprotein B enthält, weisen die einzelnen Lipoproteinklassen mehrere strukturell verschiedene Apolipoproteinklassen auf.

### Lipoprotein-Transport

Cholesterin wird hauptsächlich durch die beiden Lipoproteinklassen LDL und HDL transportiert. LDL sind vor allem zuständig für den Chlosterintransport zu peripheren Zellen, die spezifische Rezeptoren für die LDL besitzen. Die HDL ermöglichen und beschleunigen den Abtransport von Cholesterin aus den extrahepatischen Zellen und Gefäßwänden und führen es der Leber zu.

### Pathogenität

Hinsichtlich der Pathogenität bei Lipidstoffwechselstörungen läßt sich allgemein sagen, dass LDL-Cholesterinerhöhungen in Verbindung mit HDL-Cholesterinverminderungen die ausgeprägteste Risikoerhöhung für Arteriosklerose darstellen. In der Pathogenese spielen LDL, deren Partikel wesentlich zur Bildung atherosklerotischer Plaques beitragen, und HDL daher eine gegensätzliche Rolle. Für die Beurteilung des Infarktrisikos sind die Quotienten aus Gesamtcholesterin/HDL-Cholesterin und insbesondere LDL-Cholesterin/HDL-Cholesterin entscheident. (Auf die schützende Wirkung des HDL-Cholesterins weisen auch epidemiologische Studien (Framingham-Studie) hin.) Die Folgeerkrankungen der Arteriosklerose beinhalten neben der koronaren Herzkrankheit und peripheren arteriellen Verschlußkrankheiten insbesondere Infarkte in Herz und Gehirn (Schlaganfall).

Vermutlich verursacht oxLDL genauso wie LDL arteriosklerotische Plaques, wobei für den Körper von oxLDL die größere Gefahr ausgeht.

Aber auch bei anderen Erkrankungen scheint oxLDL eine wichtige Rolle zu spielen. Bei Patienten mit chronischem Nierenversagen und Diabetes ist die Konzentration an oxLDL-Glykoprotein-Komplexen höher als bei gesunden Patienten.

LDL wird durch die Leber und durch Makrophagen aus dem Blutkreislauf entfernt. Makrophagen sind Zellen des Fremdkörperabwehrsystems, die zur Phagozytose größerer Partikel fähig sind.

Der "Scavenger-Pathway" ist ein bekanntes Modell zur Erklärung der Aufnahme von Partikeln durch Zellen (Phagozytose). Die Aufnahme fester Partikel (Gewebstrümmer, Fremdkörper, Bakterien oder LDL-Plaques) in das Zellinnere von Phagozyten mit nachfolgendem intrazellulären Abbau erfolgt durch Phagozytose. Die zu Phagozytose befähigten Zellen werden auch als Fresszellen bezeichnet und bestehen überwiegend aus Gewebsmakrophagen sowie aus mobilen Blutmonozyten.

Bei der "Phagozytose" kommt es nach Anlagerung der Partikel an die Zellmembranen der Phagozyten durch Bindung an membranständige Fc- und Komplementrezeptoren zur Aktivierung kontraktiler Strukturen innerhalb des Zytoplasmas. Durch lokale Einstülpungen der Zellmembranen kommt es zum Einschluss der Partikel in Zytoplasmavakuolen.

Die sog. Abräum-(Scavenger-)Phagozyten findet man im Lymphknoten in und entlang der zur Medulla (Mark) gehörenden Faserstränge. Während der Lymphpassage vom afferenten zum efferenten Ende des Lymphknotens werden partikuläre Antigene durch die zur Phagozytose befähigten Zellen entfernt.

Darüberhinaus ist bekannt, dass die Adhärenz an phagozytierende Zellen, wie polymorphkemige Leukozyten und Makrophagen, durch die Anhaftung von Immunoglobulinen (Ig) an die Oberfläche von Bakterien (und anderen Antigenen) vergrößert ist. Es wird vermutet, dass die gesteigerte Adhärenz durch Anlagerung des Fc-Anteils des Immunoglobulins an die Fc-Rezeptoren der Phagozyten bewirkt wird. Nach dem "Schmackhaftmachen der Antigene durch Anhaftung von (od Bindung von) Antikörpern" wird der Komplex aus Antigen und Antikörpern von den phagozytierenden Zellen schneller aufgenommen und verdaut. Die Beschichtung der Antikörperoberfläche mit Immunoglobulinen wird auch als opsonin-bedingte (Fc) Adhärenz bezeichnet und spielt eine wichtige Rolle bei der Immunabwehr.

Die an die Oberfläche von Bakterienzellen bindenden Antikörper sind in der Lage, bestimmte Komponenten der extrazellulären Flüssigkeiten zu fixieren. Im Oberbegriff werden diese Komponenten als "Komplement" bezeichnet. Tierversuche zeigten, dass die Phagozytose von mit Antikörpern beschichteten Zellen bei jenen Tieren mit Komplementmangel verzögert ist. Somit liegt nahe, dass es bei der Opsonierung einen Synergismus zwischen Antikörpern und Komplement gibt.

Getz GS, 2001:"The first human monoclonal antibody to oxidized LDL", Arterioscler Thromb Vasc Biol volume 21, pages 1254-1255, beschreibt einen humanen monoklonalen Antikörper, der spezifisch oxidiertes LDL, aber nicht natives LDL bindet, und diskutiert seine möglichen diagnostischen und therapeutischen Anwendungen im Zusammenhang mit Arteriosklerose.

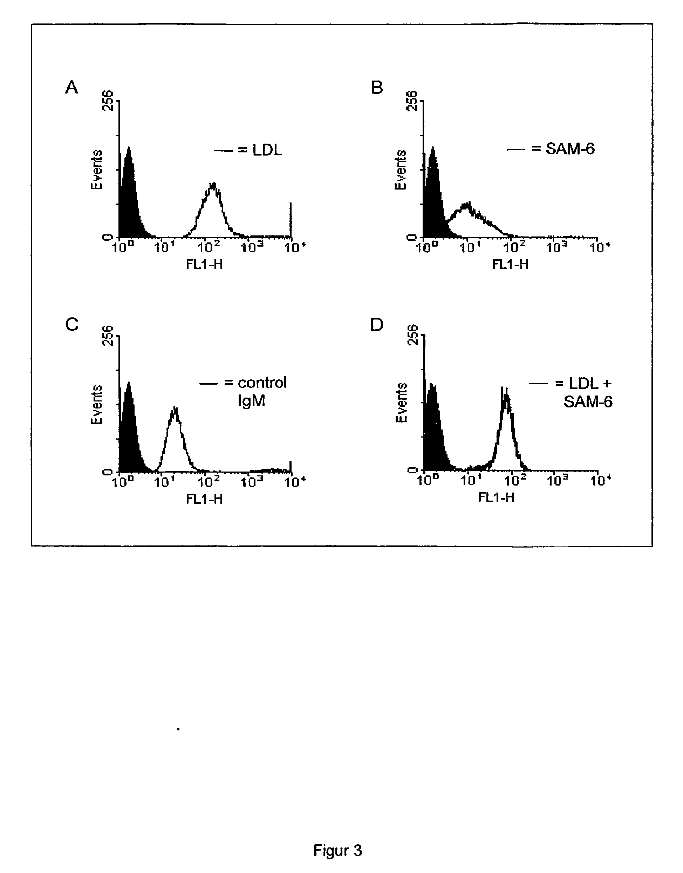

Die WO 03/048321 A2 offenbart einen Hybrid-Antikörper bzw. ein Fragment davon. Insbesondere werden in Abbildung 3a einzelne Aminosäuresequenzen von V_{L}-Ketten von Antikörpern gezeigt.

### Beschreibung der Erfindung

Die Wirkung der aus dem Stand der Technik bekannten Arzneimittel zur Senkung des LDL-Cholesterins beruht auf der Hemmung des Schlüsselenzyms der Cholesterinsynthese (CSE). Als Cholesterinsynthesehemmer ist beispielsweise eine unter dem Handelsnamen Lipobay vermarktet Substanz bekannt geworden. Die Nebenwirkungen der CSE-Hemmer allgemein sind erheblich und schließen u.a. gastrointestinale Störungen, Schlafstörungen, Schwindel, Sehstörungen, allergische Reaktionen und Haarausfall ein. Lediglich im Versuchsstadium, und zwar nur bei schwerer familiärer Hypercholesterinämie, ist ein Ansatz der somatischen Gentherapie, der die Übertragung des Gens für den LDL-Rezeptor auf autologe Leberzellen zum Inhalt hat.

Ein weitgehend nebenwirkungsfreier Stoff, der als Fettsenker wirkt, ist bislang nicht auf dem Markt. Insbesondere sind Antikörper, die eine verstärkte intrazelluläre Akkumulation von Lipoproteinen induzieren, bislang nicht bekannt. Obwohl die schädigende Rolle von Lipoproteinen bei Nierenerkrankungen (Lipid-induzierte Schädigung von Zellen des Glomerulus-Filtrations-Apparates der Niere) bekannt ist, gibt es noch keinen auf Antikörper basierenden Therapie-Ansatz von Nieren Erkrankungen, insbesondere der Glomerulonekrose.

Die Aufgabe der Erfindung besteht in der Generierung eines neuen Stoffes bzw. einer neuen Stoffklasse zur Herstellung eines Arzneimittels zur Verringerung des LDL-Cholesterins bei Mensch und Tier mit dem vorteilhaften Ziel der Verringerung des Infarktrisikos.

Zur Lösung der Aufgabe wird ein gereinigter Antikörper vorgeschlagen mit einer Aminosäuresequenz der variablen Region der leichten Kette (V_{L}), die zu mindestens 90 % identisch ist mit der Aminosäuresequenz von SEQ ID NO:1 und einer Aminosäuresequenz der variablen Region der schweren Kette (V_{H}), die zu mindestens 90 % identisch ist SEQ ID NO:3 und der Low Density Lipoprotein (LDL), insbesondere LDL-Cholesterin, bindet.

Ferner wird die Aufgabe gelöst durch einen Antikörper, enthaltend die Complementarity-determining regions (CDR), die in der variablen Region der schweren Kette (V_{H}) die Aminosäuresequenzen Ser-Tyr-Ala-Met-His (CDR1), Val-Ile-Ser-Tyr-Asp-Gly-Ser-Asn-Lys-Tyr-Tyr-Ala-Asp-Ser-Val-Lys-Gly (CDR2) und Asp-Arg-Leu-Ala-Val-Ala-Gly-Lys-Thr-Phe-Asp-Tyr (CDR3) von SEQ ID NO:3 aufweisen und die in der variablen Region der leichten Kette die Aminosäuresequenzen Ser-Gly-Asp-Lys-Leu-Gly-Asp-Lys-Tyr-Ala-Cys (CDR1), Gln-Asp-Ser-Lys-Arg-Pro-Ser (CDR2) und Gln-Ala-TrpAsp-Ser-Ser-Ile-Val-Val (CDR3) der SEQ ID NO:1 der variablen Region der leichten Kette (V_{L}) aufweisen, und wobei der Antikörper Low Density Lipoprotein (LDL), insbesondere LDL-Cholesterin, bindet.

Daneben wird zur Lösung der Aufgabe ein Verfahren zur Erzeugung des erfindungsgemäßen Antikörpers durch die Hybridomtechnologie vorgeschlagen, bei dem die Hybridomzellen durch Fusion von Heteromyelomazellen HAB-1 und ihren Subklonen mit aus menschlicher/n Milz, Lymphknoten oder Blut entnommenen B-Lymphozythen gewonnen wurden.

Der Kern der Erfindung besteht in der überraschend gemachten Beobachtung, dass ein gereinigter Antikörper, dessen Sequenz der leichten (V_{L}) und schweren Kette (V_{H}) eines humanen monoklonalen Antikörpers (SAM-6.1 0) entspricht, die Senkung des low density lipoproteins (LDL) bewirkt. Die Entdeckung dieser Eigenschaft, die den Einsatz des Antikörpers in entsprechender pharmazeutischer Formulierung als Fettsenker nahelegt, wurde im Rahmen der biochemischen Charakterisierung des Antikörpers gemacht. Vorteilhafterweise ist die Bindung des erfindungsgemäßen Antikörpers an LDL und an VLDL, den Vorläufern der LDL, stärker ist als die Bindung an HDL. Aufgrund dieser Eigenschaft führt der erfindungsgemäße Antikörper zu einem geringen Wert für den jeweiligen Quotienten aus LDL/HDL und minimiert somit das Infarktrisiko.

Die spezifische Bindung des Antikörpers an Low Density Lipoproteine (LDL), bzw. LDL-Cholesterin wurde experimentell durch die ELISA-Methode nachgewiesen. Im gleichen Experiment konnte auch gezeigt werden, dass die Bindung des erfindungsgemäßen Stoffes an High Density Lipoproteine (HDL) schwach ist.

In einer Weiterbildung des erfindungsgemäßen Antikörpers handelt es sich um einen humanen monoklonalen Antikörper.

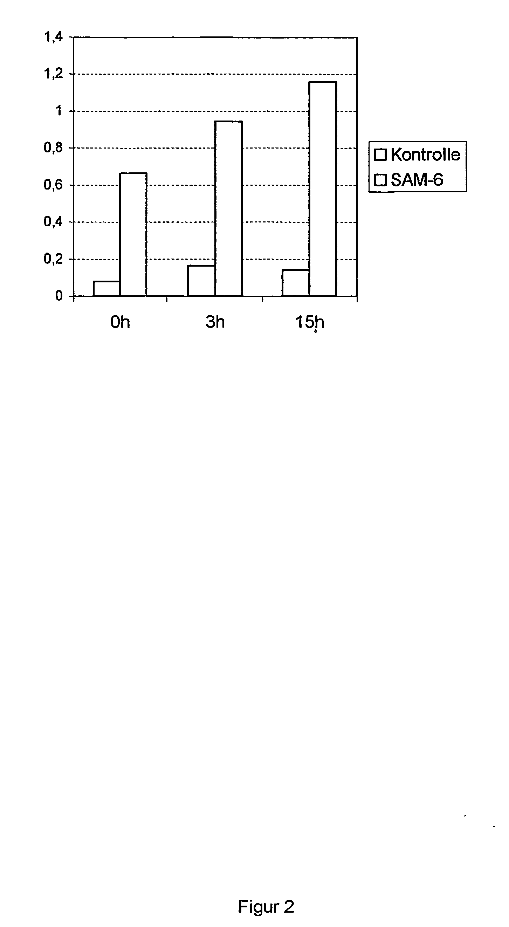

Die Complementartity-Determining Regionen (CDRs) der variablen Region der leichten Kette des Antikörpers (V_{L}) beinhalten die Aminosäuresequenzen Ser-Gly-Asp-Lys-Leu-Gly-Asp-Lys-Tyr-Ala-Cys (CDR1), Gln-Asp-Ser-Lys-Arg-Pro-Ser (CDR2) und Gln-Ala-Trp-Asp-Ser-Ser-Ile-Val-Val (CDR3) der SEQ ID NO:1; siehe auch Figur 2.

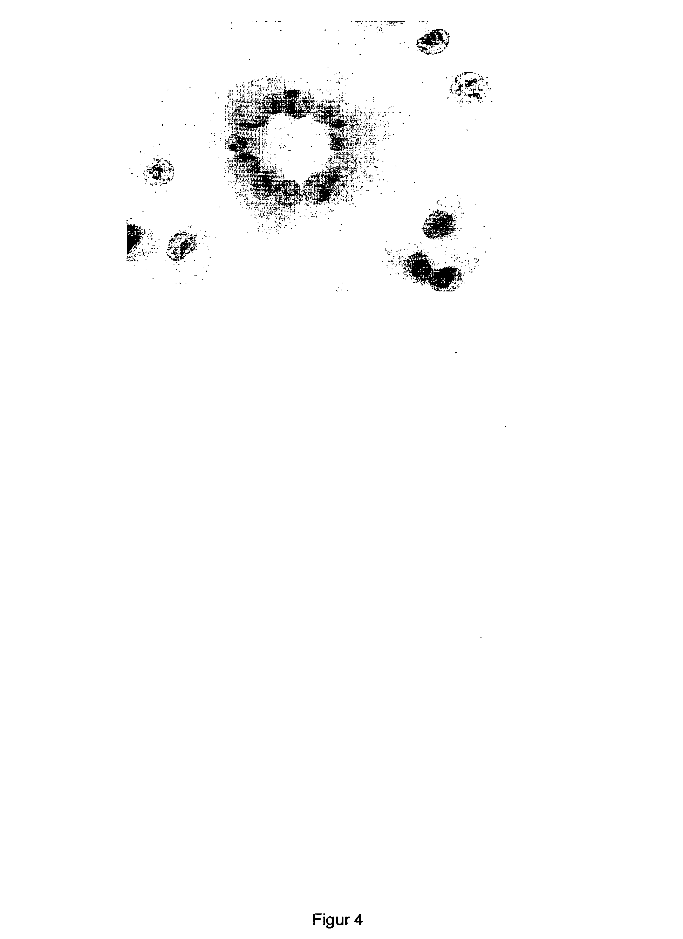

Die complementarity-determining regions (CDRs)des Antikörpers oder funktionellen Fragments beinhalten Aminosäuresequenzen, die im wesentlichen identisch sind mit Ser-Tyr-Ala-Met-His (CDR1), Val-Ile-Ser-Tyr-Asp-Gly-Ser-Asn-Lys-Tyr-Tyr-Ala-Asp-Ser-Val-Lys-Gly (CDR2) und Asp-Arg-Leu-Ala-Val-Ala-Gly-Lys-Thr-Phe-Asp-Tyr (CDR3) der SEQ ID NO 3 der variablen Region leichten Kette (V_{H}); siehe auch Figur 4.

Als "im wesentlichen identisch" wird eine Aminosäuresequenz oder eine Nukleinsäuresequenz bezeichnet, die zumindest oder 90 % mit der als Referenz angegebenen Aminosäurensequenz (SEQ ID IO: 1 und 3) oder mit der Nukleinsäurensequenz (SEQ ID NO; 2 und 4) aufweist. In einer Weiterbildung der Aminosäuresequenz bzw. der Nukleinsäurensequenz sind mindestens 95 %, 98 %, 99 % oder 100 % Identität im Vergleich zu den angegebenen Referenzen nachweisbar.

Der erfindungsgemäße Antikörper ist generierbar durch ein Verfahren, das unter dem Namen Hypridoma-Technik (Köhler, Millstein, Nature, 1975, Vol. 256,495) bekannt ist und die Isolation von monoklonalen Antikörpern ermöglicht. Es beruht auf der in vitro-Gewinnung von zellulären Hybriden die durch Zellfusion von normalen Lymphozyten mit unbegrenzt lebens- und teilungsfähigen Myelomzellen (z.B. HAB-1) gewonnen werden. Die hierbei erzeugten Hybridom-Zellen weisen Eigenschaften beider Elternzellen auf. Dem entsprechend besitzen sie die Fähigkeit der Lymphozyten, Antikörper zu produzieren (z.B. SAM-6.10) und die Fähigkeit der Myelomzelle zur unbegrenzten Teilung und damit zur Produktion der Antikörper in großen Mengen. Jede aus der Fusion resultierende Hybridzelle stellt monoklonale Antikörper her deren Spezifität von der ursprünglichen

Lymphozytenzelle bestimmt wird. Die Hybridomzellen werden vermehrt und dann diejenigen selektiert, welche Antikörper der gewünschten Spezifität produzieren. Die Kultivierung dieser Auswahl und deren Isolierung führt zu hochspezifisch reagierenden Antikörpern, welche nur mit einer bestimmten antigenen Determinante reagieren.

Der Nachweis der deutlichen Senkung des low density lipoprotein (LDL)-Spiegels (bzw. des LDL-Cholesterin Spiegels) im Blutplasma wurde im Tierexperiment bestätigt, ohne dass sich der HDL-Spiegel im nachweisbaren Bereich senkt. Wesentlich dabei ist, dass die Vitalfunktionen der Tiere während der Gabe des Antikörpers nicht beeinträchtigt werden, so dass die erfindungsgemäße Substanz bislang als Nebenwirkungsfrei bezeichnet werden kann. (Die Wirkungsweise des erfindungsgemäßen Antikörpers könnte sich in Analogie zum Mechanismus des bekannten Scavenger-Pathways erklären lassen.)

Eine weiteres Indikationsgebiet für den Einsatz des erfindungsgemäßen Medikamentes ist die Behandlung von Nierenerkrankungen, insbesondere die Glomerulonekrose (Glomerulosklerose).

Es liegt im Rahmen der Erfindung, dass das erfindungsgemäße Polypeptid zur Herstellung des Arzneimittels bevorzugt in gereinigter Form eingesetzt wird, wobei zur Reinigung sämtliche dem Fachmann bekannten Verfahren (z.B. Affinitätschromatographie, Gelfiltration) in Frage kommen. Als Indikation des erfindungsgemäßen Stoffes steht des fettsenkende Wirkung im Vordergrund, wobei insbesondere die selektive Senkung von LDL bzw. LDL-Cholesterin hervorzuheben ist. Aufgrund der Eigenschaft, LDL stärker zu binden als HDL,bewirkt das erfindungsgemäße Polypeptid einen geringen Wert für den Quotienten aus LDL/HDL und minimiert somit das Infarktrisiko.

Die Hilfs- und Trägerstoffe zur Herstellung eines Arzneimittels sind dem Fachmann bekannt und können nach gängiger Praxis hergestellt werden (siehe Remington: The Science and Practice of Pharmacy (20th ed.), ed. A.R. Gennaro, Lippincott Williams & Wilkins, 2000 and Encyclopedia of Pharmaceutical Technology, eds. J. Swarbrick and J. C. Boylan, 1988-1999, Marcel Dekker, New York).

### Material und Methoden

### Immortalisierung von Lymphozyten und Primärtestung der Antikörper

Zur Immortalisierung werden die Lymphozyten mit dem Heteromyelom HAB-1 (Faller et al., 1990) nach Standardprotokoll fusioniert und kultiviert. Kurz zusammengefasst, Lymphozyten werden mit HAB-1 Zellen mittels PEG verschmolzen. Die Triome werden auf vier 24-Lochplatten ausgesät. Die durchschnittliche Wachstumsfrequenz beträgt 80-90%, 50% der wachsenden Klone sezernieren Immunglobuline. Die erste Austestung der sezernierten humanen monoklonalen Antikörper erfolgt im ELISA, um den Isotyp zu ermitteln. Nachfolgend können die humanen monoklonalen Antikörper immunhistochemisch, genetisch, biochemisch und molekularbiologisch Analysiert werden.

Benötigte Medien:
● RPMI 1640 (Firma PAA) ohne Zusätze
   RPMI 1640 mit HAT-Zusatz (HAT-Supplement, Firma PAA) sowie 10% FCS, 1% Glutamin und 1% Penicillin/Streptomycin
● HAB-1 (Fusionspartner) zweimal mit RPMI ohne Zusätze waschen
● zentrifugieren 5 min bei 1500U/min
● eingefrorene Lymphozyten (aus Milz, Lymphknoten oder Blut) auftauen und zweimal mit RPMI ohne Zusätze waschen, ebenfalls zentrifugieren
● beide Pellets jeweils in 10 ml RPMI ohne Zusatz aufnehmen und in der Neubauer-Zählkammer zählen
● im Verhältnis von 1:2 - 1:3, Hab-1 zu Lymphozyten, fusionieren
● die Zellpellets nach dem zweiten Waschvorgang zusammen geben, mischen und 8 min bei 1500 U/Min zentrifugieren
● das zuvor bei 37°C aufgewärmte PEG (Polyethylene Glycol 1500, Firma Roche) vorsichtig tröpfelweise auf das Pellet unter leicht rotierenden Bewegungen des 50 ml Röhrchens laufen lassen
● leicht resuspendieren und dann genau 90 sek. im Wasserbad bei 37°C rotieren lassen
● danach wir das PEG mit RPMI ohne Zusätze herausrausgewaschen (zwei volle 10er Pipetten)
● zentrifugieren 5 min bei 1500 U/min
● 24 Well-Platten ausplattieren mit 1ml pro Well RPMI mit HAT-Zusatz (HAT= Hypoxanthin, Aminopterin, Thymidin)
● das Pellet lösen in RPMI mit HAT-Zusatz
● jeweils einen halben ml der Zellen in ein 24 Well pipettieren
● Fusionsplatten in den Brutschrank stellen
● wöchentlich Mediumwechsel mit RPMI mit HAT-Zusatz

### Reinigung des Antikörpers SAM-6.10

### Reinigung von Kulturüberstand durch Kationenaustauschchromatographie über FPLC

Die den IgM-Antikörper SAM-6.10 produzierenden Hybridomzellen wurden hierzu in einem speziellen serumfreien Zellkulturmedium (AIMV-Medium, Gibco) herangezogen und der Gehalt an IgM im Kulturüberstand nephelometrisch bestimmt. Zur Aufreinigung wurde der Kulturüberstand auf einen pH-Wert von auf 5,9 eingestellt und die Lösung filtriert. Zur Bindung wurde eine spezielle Kationen-Säule (HiTrap™ SP FF column, 5 ml, Amersham Bioscience) verwendet. Die Säule wurde zu Beginn der Reinigung mit filtriertem Puffer A (20 mM Phosphatpuffer, pH 5,9) äquilibriert. Anschließend wurde der auf Eis gekühlte Kulturüberstand mit einer Flussrate von 1 ml/min auf die Säule aufgetragen. Nach dem Auftrag des Überstandes wurde die Säule für 20 min und einer Flussrate von 2 ml/min mit Puffer A bis zu einer Basislinienkonstanz gewaschen um alle nicht gebundenen Proteine zu entfernen. Anschließend wurde der an die Säule gebundenen Antikörper durch Beimischung von Puffer B (20 mM Phosphatpuffer, 1 M NaCl, pH 8,0) eluiert und in Fraktionen gesammelt. Der Gehalt an SAM-6.10 Antikörper (IgM) in den einzelnen Fraktionen wurde im Folgenden nephelometrisch bestimmt und die Reinheit und Intaktheit des gereinigten Antikörpers über SDS-PAGE und Western-Blot Analyse überprüft.

### Referenzmessung von oxLDL

Die Messung erfolgt mit Hilfe des Test-Kits Oxidised LDL ELISA von der Firma Mercodia, Uppsala, Schweden. Test-Prinzip: Der ELISA für oxidiertes LDL ist ein enzymatischer, zweiseitiger Immunoassay mit einer Festphase. Er basiert auf der direkten Sandwich-Technik, bei der zwei monoklonale Antikörper gegen ein bestimmtes Antigen auf oxidiertem ApolipoproteinB ausgerichtet werden. Während der Inkubation reagiert oxidiertes LDL in der Probe mit anti-oxidiertem LDL-Antikörpern, die an die Vertiefungen in der Mikrotiterplatte gebunden sind. Nach dem Waschen, wobei die nicht-reaktiven Bestandteile der Probe entfernt werden, erkennt ein peroxidasekonjugierter anti-human Apolipoprotein B Antikörper das an der festen Phase gebundene oxidierte LDL. Nach einer zweiten Inkubation und einem Waschvorgang, der die mit dem ungebundenen Enzym markierten Antikörper entfernt, wird das Konjugat durch Reaktion mit 3, 3', 5, 5'-Tetramethylbenzidin (TMB) nachgewiesen. Die Reaktion wird durch Zugabe von Säure gestoppt, um einen kolorimetrischen Endpunkt zu definieren der bei 450 nm spektrophotometrisch abgelesen wird.

### Referenzmessung der Bindung von SAM-6 an oxLDL

Eine ELISA-Platte (Becton Dickinson Labware Europ, Frankreich) wurde mit den unterschiedlich stark oxidierten LDL Fraktionen über Nacht bei 4°C inkubiert. Die unspezifischen Bindungsstellen wurden mit RPMI-1640 Medium welches mit 10% FCS versetzt wurde für 1 h geblockt. Anschließend wurde die Platte mit 60µg/ml SAM-6 Antikörper für eine Stunde bei 37°C inkubiert. Nach dreimaligem Waschen mit PBS wurde die ELISA-Platte für 1 h mit HRP-gekoppeltem Zweitantikörper (rabbit anti human IgM, Dako, Hamburg, Deutschland, 1:1000 in PBS) inkubiert. Danach wurde die Platte nochmals mit PBS und Citratpuffer gewaschen, mit OPD (DakoCytomation, Glostrup, Dänemark) versetzt und der Farbumschlag bei 490 nm mit einem ELISA-reader gemessen.

### Durchflusszytometrie (FACS Analyse)

Die verwendeten adhärenten Zellen wurden durch Behandlung mit Trypsin/EDTA vom Boden der Kulturflaschen abgelöst. Die Reaktion wurde mit 10 ml RPMI-1640 Medium (+ Supplemente) abgestoppt und die Zellen mit 1000 x g für 5 min pelletiert. Die Zellen wurden zweimal mit PBS gewaschen, in FACS-Puffer (PBS, 0,01% Na-Azid) aufgenommen und zur Rekonstitution der Zellmembranen für 30 min auf Eis gestellt. Anschließend wurden die Zellen auf eine Dichte von 1 x 10⁶ Zellen/ml eingestellt und je 200 µl der Zellsuspensionen in FACS-Reaktionsgefäße überführt, sodass pro Röhrchen eine Zellzahl von 2 x 10⁵ Zellen vorlag. Die Zellen wurden für 5 min bei 4°C und 1400 rpm pelletiert, die Pellets resuspendiert und mit den Primär-Antikörpern für 15 min auf Eis inkubiert. Als Primär-Antikörper wurden 100 µg/ml SAM-6 Antikörper in FACS-Puffer (Gesamtvolumen 200 µl) bzw. 100mg/ml LDL verwendet. Als Isotyp-Kontrolle fungierten 100 µg/ml Chrompure human IgM. Alternativ wurden die Zellen mit LDL für 30 min vorinkubiert und anschließend für 15 min 100µg/ml SAM-6 Antikörper zugesetzt.

Nach der Inkubation wurden die Zellen abzentrifugiert, der Überstand verworfen und die Pellets mit 500 µl kaltem FACS-Puffer gewaschen. Danach erfolgte eine 15minütige lichtgeschützte Inkubation mit dem FITC-gekoppelten Sekundär-Antikörper (Rabbit anti human IgM, FITC-gekoppelt, 1:50 in FACS-Puffer für SAM-6 bzw. Chrompure human IgM). Nach einem erneuten Waschgang wurden die Zellen in 200 µl kaltem FACS-Puffer aufgenommen und bis zur Messung lichtgeschützt auf Eis aufbewahrt. Die Messung erfolgte mittels eines Durchflußzytometers (FACScan; Becton Dickinson, USA).

### Tierexperimente zum Nachweis der in-vivo Wirkung des Antikörpers SAM-6.10

**Experiment 1:** 500µg gereinigter SAM-6.10 Antikörper wurden intra peritoneal injiziert. Die LDL Konzentration im Blutserum wurde nach 2 Tagen gemessen (Methode siehe unten).
**Experiment 2:** wie oben
**Experiment 3:** 1 mg gereinigter SAM-6.10 Antikörper wurden intrapereitoneal injiziert. Die LDL Konzentration im Blutserum wurde nach 14 Tagen gemessen.

Kontrolle A: Normalwerte Kontrollmaus
Kontrolle B: Normalwerte Kontrollmaus

Die Serumkonzentration von LDL ist bei den mit SAM-6.10 behandelten Mäusen deutlich reduziert (siehe Figur 10 und 11).

### Toxizität

500µg bzw. 1 mg gereinigter SAM-6.10 Antikörper wurde Mäusen intraperitoneal injiziert.
● keine akute Toxizität
● keine latente Toxizität (Zeitraum 3 Monate).

Die Organe der getöteten Mäuse aus Experiment 1, 2 und 3 (siehe oben) wurden entnommen und untersucht: Leber, Lunge, Herz, Milz, Dünndarm, Dickdarm, Nieren, Magen und Gehirn wiesen keine morphologischen Veränderungen auf. Darüber hinaus wurden die Organe immunhistochemisch auf eventuelle Lipideinlagerungen untersucht. Die Färbung mit Sudan III zeigte in keinem Organ eine Einlagerung von Lipiden.

Die Organe der getöteten Mäuse wurden in Formalin fixiert und in Paraffin eingebettet. Die Färbung erfolgte mit dem Farbstoff Sudan III nach folgendem Protokoll:

### Sudan III-Färbung auf Parafffinschnitten/ auf Makrophagen

Entparaffinierung:
● Xylol 1 5 min
● Xylol 2 5 min
● 100% Ethanol 1 5 min
● 100% Ethanol 2 5 min
● Methanol 70ml
   + H₂O₂ 500µl 5 min
● 90% Ethanol 1 3 min
● 90% Ethanol 2 3 min
● 80% Ethanol 1 3 min
● 80% Ethanol 2 3min
● 70% Ethanol 1 3 min
● 70% Ethanol 2 3 min
   ● Schnitte in PBS stellen
   ● Schnitte 15 min mit Sudan III inkubieren
   ● waschen mit Aqua dest.
   ● 1 x in 60% Isopropanol eintauchen
   ● waschen mit Aqua dest.
   ● Gegenfärbung mit Hämalaun für 6 min
   ● Schnitte 10 min wässern, waschen mit Aqua dest und mit Glyceringelatine eindeckeln

Für die Sudan III Färbung von Makrophagen werden die adherenten Makrophagen auf Objekträgern angezüchtet und dann mit den entsprechenden Agenzien behandelt. Die Färbung wird wie folgt durchgeführt:
● Fixierung der Zellen in 60% Isopropanol (6 min)
● 20 min mit Sudan III inkubieren
● waschen mit Aqua dest.
● Gegenfärbung mit Hämalaun für 6 min
● Schnitte 10 min wässern, waschen mit Aqua dest und mit Glyceringelatine eindeckeln

### Bestimmung von Lipiden in Blutproben

Die Messung der verschiedenen Lipide im Blutserum wurde automatisch mit dem MODULAR D P800 Gerät (Roche) vorgenommen.

Die Bestimmung des LDL-Cholesterinwertes erfolgte durch eine enzymatische, kolorimetrische Methode (CHOD/PAP) ohne Probenvorbehandlung.

### Testprinzip zur Bestimmung der Lipidkonzentration im Serum:

HDL, VLDL und Chylomikronen werden von einem Detergenz 1 spezifisch hydrolysiert. Das in diesen Lipoproteinen freigesetzte Cholesterin reagiert sofort durch die enzymatische Wirkung der Cholesterinesterase (CE) und Cholesterinoxidase (CHOD) und es entsteht Wasserstoffperoxid. Dies bildet mit 4-Aminoantipyridin in Anwesenheit einer Peroxidase (POD) ein farbloses Produkt. Während dieses Schrittes bleiben die LDL-Partikel intakt. Die Reaktion von LDL-Cholesterin wird durch Zugabe von Detergenz 2 und der Kupplungssubstanz N,N-bis(4-Sulfobutyl)-m-toluidin (DSBmT) ausgelöst. Das zweite Detergenz setzt Cholesterin in den LDL-Partikeln frei. In der enzymatischen Reaktion wird in Anwesenheit der Kupplungssubstanz ein Farbstoff gebildet. Die Intensität des gebildeten roten Chinoniminfarbstoffs ist direkt proportional zur LDL-Cholesterin-Konzentration. Sie wird durch Messung der Extinktionszunahme bei 552 nm bestimmt.

### ELISA (LDL/HDL)

- ELISA-Platte vorbeschichten mit LDL (Lipoprotein Low Densi tyfrom Human Plasma, Firma Sigma, 10µg/ml in PBS) oder HDL (Human HDL, Firma Chemicon 10µg/ml in PBS) → 50 µl pro well
- Platte abdecken und über Nacht bei 4°C lagern
- am nächsten Tag Platte 2 x mit PBS waschen
- 100 µl RPMI in jedes well pipettieren. und für 1 h bei RT inkubieren
- danach 2 x mit PBS waschen
- 50 µl der jeweiligen Positivkontrollen in je 2 wells pipettieren (Doppelbestimmung)
   Positivkontrolle: Mab to human LDL Mouse IgG2a 1:1000 in PBS
- daneben 50 µl RPMI als Negativkontrolle (Doppelbestimmung)
- 50 µl der Proben (Überstand SAM6.10) (Doppelbestimmung) nebeneinander pipettieren
- 1 h im Brutschrank inkubieren
- 2 x mit PBS waschen
- 2 x mit PBS/0,05% Tween waschen
- 2 x mit PBS waschen
- 50 µl der jeweiligen 2. AK (Peroxidase konjugiert.) pipettieren:
   rabbit anti human IgM 1:1000 in PBS/0,05%Tween (für SAM-6.10)
   rabbit anti Mouse IgGs 1:1000 mit PBS Tween (für Positivkontrolle LDL)
- 1 h im Brutschrank inkubieren
- 2 x mit PBS waschen
- 1 x mit PBS/0,05% Tween waschen
- 2 x mit PBS waschen
- 2 x mit Citratpuffer waschen
- zum Auswerten: OPD Tablette (Dako, Hamburg) in Citratpuffer lösen + H₂O₂ (3 ml Citratpuffer + eine Tabl. + 5 µl H₂O₂)
- 50 µl Farbstoff in jedes Well pipettieren
- bei positiver Reaktion (gelbe Färbung) mit 10 µl 3 M H₂SO₄ stoppen

### Erläuterung der Figuren

### Sequence listing

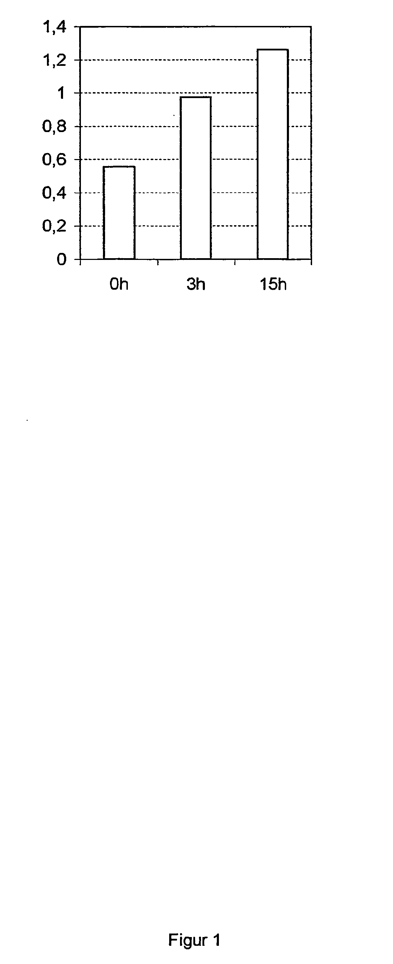

Figur 1 zeigt die Aminosäuresequenzen (SEQ ID NO 1) der variablen Region der leichten Kette (V_{L}).
Figur 2 zeigt die Nucleotidsäuresequenz (SEQ ID NO 2) der variablen Region der leichten Kette (V_{L}). Die complementarity-determining regions (CDR) sind durch Querstriche gekennzeichnet und im wesentlichen identisch mit den Nucleotiden 67-99 (CDR1), 145-165 (CDR2) und 262-288 (CDR3) von SEQ ID NO 2.
Figur 3 zeigt die Aminosäuresequenzen (SEQ ID NO 3) der variablen Region der schweren Kette (V_{H}).
Figur 4 zeigt die Nucleotidsäuresequenz (SEQ ID NO 4) der variablen Region der schweren Kette (V_{H}). Die complementarity-determining regions (CDR) sind durch Querstriche gekennzeichnet und im wesentlichen identisch mit den Nucleotiden 91-105 (CDR1), 148-198 (CDR2) und 295-330 (CDR3) von SEQ ID NO 4.

### Zellbiologische Experimente

Die nachfolgende erklärten Figuren sollen die Erfindung nicht einschränken, sondern nur erläutern und deren Ausführbarkeit beispielhaft belegen.

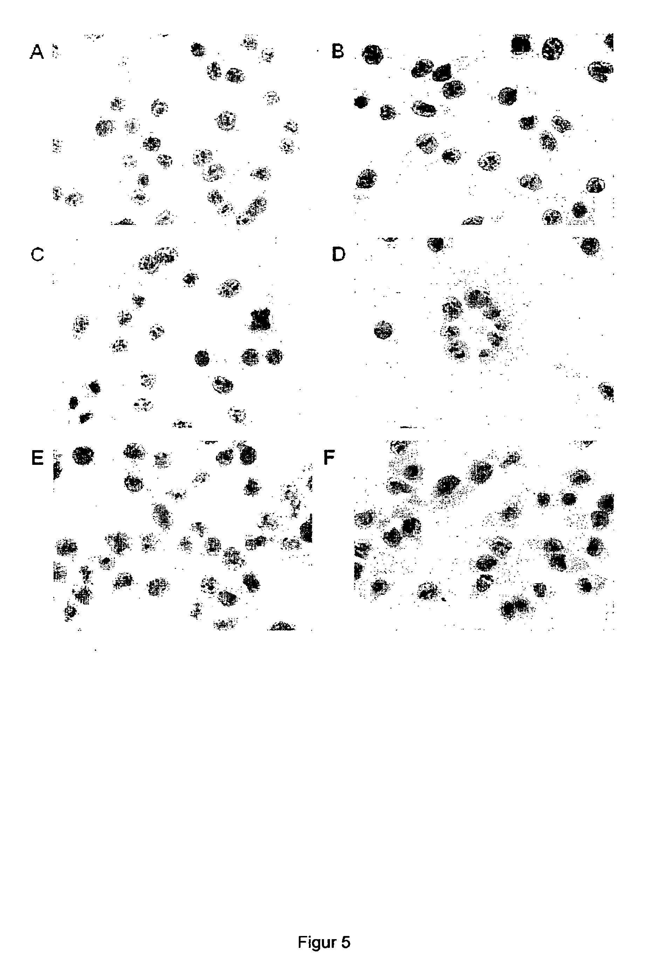

Figur 5 zeigt die Referenzmessung von oxLDL in Abhängigkeit von der Inkubationdauer mit einer Kupfersulfatlösung. Im Versuch wurde LDL (Sigma, Taufkirchen, Deutschland) wurde für 3 bzw. 15h mittels Inkubation mit 20µM CuSO₄ oxidiert. Mit dem Mercodia Oxidised LDL ELISA wurde, der Gebrauchsanweisung folgend, die Menge an oxidiertem LDL bestimmt. Deutlich zu erkennen ist, dass die Menge an oxidiertem LDL mit steigender Inkubationsdauer zunimmt, wobei auch jene LDL Fraktion, welche nicht mit Kupferionen behandelt wurde, zum Teil schon in oxidierter Form vorliegt. Nach 15 stündiger Inkubation ist der Anteil an oxidiertem LDL etwa verdoppelt.

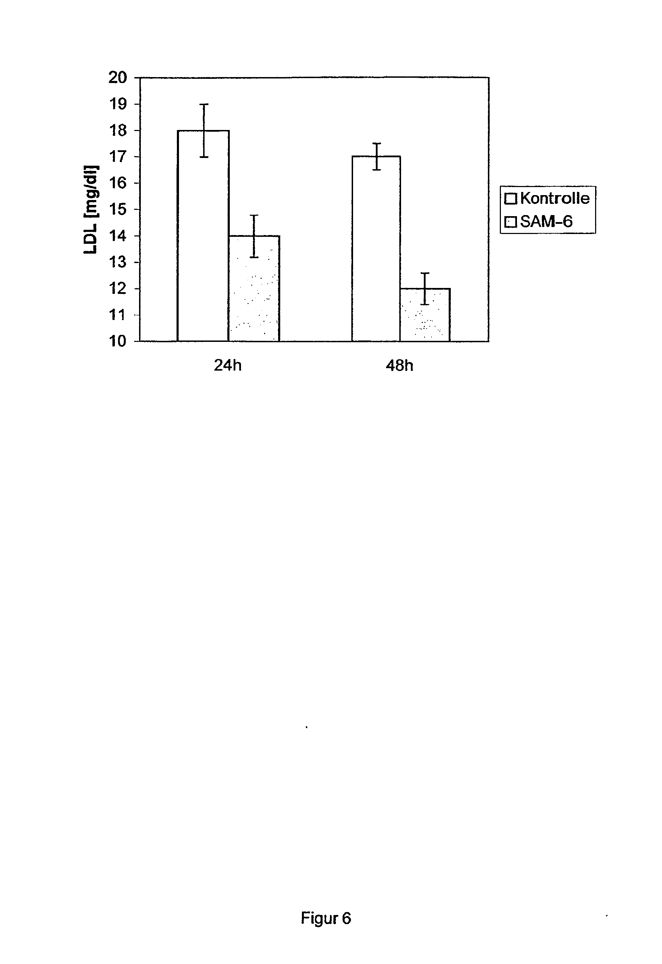

Figur 6 zeigt den Referenznachwei, der Bindung von SAM-6 an oxLDL. Für den Nachweis der Bindung von SAM-6 an oxLDL mittels ELISA Bindungsassay wurden die ELISA Platte mit unterschiedlich stark oxidierten LDL Fraktionen vorbeschichtet, bevor der Erstantikörper SAM-6 und der zu Detektionszwecken erforderliche Zweitantikörper anti-human IgM hinzugegeben wurde. Das Ergebnis zeigt, dass je mehr LDL in seiner oxidierten Form vorliegt, desto stärker bindet der erfindungsgemäße Antikörper SAM-6.

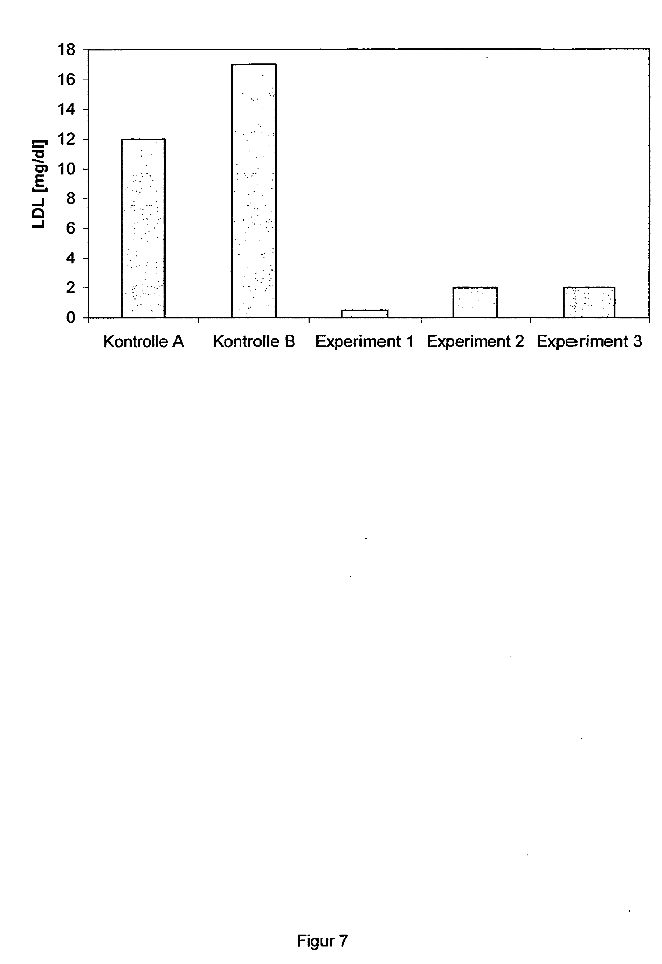

Figur 7 zeigt das Ergebnis einer FACS Analyse. Bei den dafür verwendeten Zellen handelt es sich um Zellen der Maus-Makrophagen Zellinie P388D1(IL-1) (DSMZ Accession No. ACC 288). Figur 7A zeigt die Bindung von LDL an Macrophagen. Figur 7B belegt, dass auch der humane monoklonale Antikörper SAM-6 an Macrophagen bindet. Mit dem Nachweis der Bindung eines Kontroll IgM an Macrophagen in Figur 7C wird gezeigt, dass die Macrophagen µ-Rezeptoren besitzen. Mit der Rechtsverschiebung des Signals in Figur 7D wird gezeigt, dass eine gleichzeitige Inkubation von LDL und SAM-6 eine vermehrte Bindung von SAM-6 an die Zellen bewirkt.

Die Figuren 8 und 9 zeigt das Ergebnis der Sudan III Färbung für die Zellen der Maus-Makrophagen Zellinie P388D1(IL-1) verwendet wurden.

In Figur 8 sind Zellen gezeigt, die für 48h entweder mit SAM-6 oder einem IgM Kontrollantikörper inkubiert und anschließend einer Sudan III Färbung unterzogen wurden. Die mit dem Antikörper SAM-6 inkubierten Zellen zeigen aufgrund der Rotfärbung eine deutliche Einlagerung von Neutralfetten. Die mit dem Kontroll-Antikörper inkubierten Zellen zeigen dagegen keine Veränderungen.

Für die in Figur 9 zeigten Färbungen wurden die Makrophagen für 24h sowohl mit als auch ohne Zusatz von FCS kultiviert. Danach wurde für weitere 24h entweder nur LDL oder nur SAM-6 beziehungsweise LDL und SAM-6 gemeinsam zugesetzt. Anschließend wurde eine Sudan III Färbung durchgeführt. Die linke Bildspalte mit den Figuren 9A, 9C und 9E zeigen Zellen, die ohne den Zusatz von FCS kultiviert wurden. Die rechte Bildspalte mit den Figuren 9B, 9D und 9F zeigen Zellen, die mit Zusatz von FCS kultiviert wurden.

Die Figuren 9A und 9B belegen, dass sowohl Makrophagen, die ohne FCS kultiviert wurden, als auch Macrophagen, die in Anwesentheit von FCS gewachsen sind, eine basale Einlagerung von Neutralfetten zeigen. Die Figur 9C belegt, dass bei Zugabe von SAM-6 zu Makrophagen, die ohne FCS kultiviert wurden, keine Fett-Einlagerung nachweisbar ist. Dagegen wird, wie in Figur 9D gezeigt, eine verstärkte Lipid-Akkumulation bei Makrophagen beobachtet, die mit FCS kultiviert wurden und denen anschliessend SAM-6 zugesetzt wurde. Die Figuren 9E und 9F zeigen, das bei einer Co-Inkubation von SAM-6 und LDL die intrazelluläre Lipid-Akkumulation sowohl bei Makrophagen, die mit FCS kultiviert wurden als auch bei denen die ohne Zusatz von FCS gewachsen sind, deutlich zu nimmt.

Figur 10 zeigt den Einfluss des Antikörpers SAM-6 auf LDL Werte *in vivo.* Im Versuch wurde den Mäusen 1 mg gereinigter SAM-6 Antikörper bzw. im Kontrollexperiement 1 mg human Chrompure IgM (Isotyp-Kontrolle) i.p. injiziert. Die LDL Konzentration im Blut wurde nach 24 und 48h gemessen. Dabei kann nach 24 und 48h Stunden bei SAM-6 behandelten Mäusen eine deutliche Reduktion von Serum LDL beobachtet werden.

Die Messung von LDL im Blutserum wurde automatisch mit dem MODULAR D P800 Gerät (Roche) vorgenommen, wobei sich die aufgetragenen Werte als Ergebnis des Diagnostik Kits "LDL-Cholesterol Direct" (Firma Roche Diagnostiks) ergeben.

Figur 11 weist ebenfalls die *in vivo* Wirkung von SAM-6.10 nach, wobei zur Errechnung der Werte eine indirekte Methode nach der Friedewald-Formel angewandt wurde. Dabei errechnet sich die Menge an LDL aus der Differenz von Gesamtcholesterin, HDL und Triglyceriden. Gemäß dieser Art der Versuchsauswertung wäre die Reduktion der Konzentration von LDL im Serum nach SAM-6.10 Behandlung noch stärker.. Diese indirekte Meßmethode muss jedoch im Vergleich zu der in Figur 10 angewandten Methode als ungenauer angesehen werden.

## Patentansprüche

1. Gereinigter Antikörper, **dadurch gekennzeichnet, dass**
- die Aminosäuresequenz der variablen Region der leichten Kette (V_{L}) zu mindestens 90 % identisch ist mit der Aminosäuresequenz von SEQ ID NO:1 und die Aminosäuresequenz der variablen Region der schweren Kette (V_{H}) zu mindestens 90 % identisch ist mit SEQ ID NO:3 und
- der Antikörper Low Density Lipoprotein (LDL), insbesondere LDL-Cholesterin, bindet.

2. Gereinigter Antikörper nach Anspruch 1, **dadurch gekennzeichnet, dass** die Aminosäuresequenz der variablen Region der leichten Kette (V_{L}) zu mindestens 95 % identisch ist mit SEQ ID NO:1 und die Aminosäuresequenz der variablen Region der schweren Kette (V_{H}) zu mindestens 95 % identisch ist mit SEQ ID NO:3.

3. Gereinigter Antikörper nach Anspruch 1 oder 2, **dadurch gekennzeichnet, dass** die Bindung des Antikörpers an Low Density Lipoproteine (LDL) und Very Low Density Lipoproteine (VLDL) stärker ist als die Bindung an High Density Lipoproteine (HDL).

4. Antikörper nach einem der vorhergehenden Ansprüche, **dadurch gekennzeichnet, dass** der Antikörper und die im menschlichen und tierischen Körper vorkommenden Low Density Lipoproteine (LDL) komplementäre Kohlenhydrat-Strukturen aufweisen.

5. Antikörper nach Anspruch 1, **dadurch gekennzeichnet, dass** die Nukleinsäuresequenz der variablen Region der leichten Kette (V_{L}) zu mindestens 90 % identisch ist mit SEQ ID NO:2 und die Nukleinsäuresequenz der variablen Region der schweren Kette (V_{H}) zu mindestens 90 % identisch ist mit SEQ ID NO:4.

6. Antikörper nach einem der Ansprüche 1 oder 2, **dadurch gekennzeichnet, dass** der Antikörper durch Nukleinsäuresequenzen kodiert ist, die identisch sind mit den Nukleotiden 67-99 (CDR1), 145-165 (CDR2) und 262-288 (CDR3) der SEQ ID NO:2 und identisch sind mit den Nukleotiden 91-105 (CDR1), 148-198 (CDR2) and 295-330 (CDR3) der SEQ: ID NO:4.

7. Antikörper, enthaltend die Complementarity-Determining Regionen (CDR), die in der schweren Kette (V_{H}) die Aminosäuresequenzen Ser-Tyr-Ala-Met-His (CDR1), Val-Ile-Ser-Tyr-Asp-Gly-Ser-Asn-Lys-Tyr-Tyr-Ala-Asp-Ser-Val-Lys-Gly (CDR2) und Asp-Arg-Leu-Ala-Val-Ala-Gly-Lys-Thr-Phe-Asp-Tyr (CDR3) von SEQ ID NO:3 aufweisen und
die in der leichten Kette die Aminosäuresequenzen (V_{L}) Ser-Gly-Asp-Lys-Leu-Gly-Asp-Lys-Tyr-Ala-Cys (CDR1), Gln-Asp-Ser-Lys-Arg-Pro-Ser (CDR2) und Gln-Ala-Trp-Asp-Ser-Ser-Ile-Val-Val (CDR3) von SEQ ID NO:1 aufweisen und wobei der Antikörper Low Density Lipoprotein (LDL), insbesondere LDL-Cholesterin, bindet.

8. Verfahren zur Erzeugung eines gereinigten Antikörpers nach einem der vorhergehenden Ansprüche durch die Hybridomtechnologie, **dadurch gekennzeichnet, dass** die Hybridomzellen durch Fusion von Heteromyelomazellen HAB-1 und ihren Subklonen mit aus menschlicher/n Milz, Lymphknoten oder Blut entnommenen B-Lymphozythen gewonnen wurden.

9. Gereinigter Antikörper nuch einem des Ansprüche 1-7, **dadurch gekennzeichnet, dass** der Antikörper durch das in Anspruch 8 beanspruchte Verfahren erzeugt werden kann.

10. Antikörper nach einem der Ansprüche 1 bis 9, **dadurch gekennzeichnet, dass** der Antikörper ein monoklonaler Antikörper ist.

11. Antikörper nach Anspruch 10, **dadurch gekennzeichnet, dass** der Antikörper ein humaner monoklonaler Antikörper ist.

12. Antikörper nach einem der Ansprüche 1 bis 11 in Kombination mit üblichen Hilfs- und/oder Trägerstoffen zur Verwendung als Arzneimittel mit fettsenkender Wirkung.

13. Antikörper nach einem der Ansprüche 1 bis 11 zur Verwendung als Arzneimittel zur Senkung von Low Density Lipoprotein (LDL) im Blut.

14. Antikörper nach einem der Ansprüche 1 bis 11 zur Verwendung als Arzneimittel zur Senkung von LDL-Cholesterin.

15. Antikörper nach einem der Ansprüche 1 bis 11 zur Verwendung Arzneimittel zur Behandlung von Nierenerkrankungen.

16. Antikörper zur Verwendung nach Anspruch 15 zur Herstellung eines Arzneimittels zur Behandlung von Glomerulonekrose.

## Claims

1. A purified antibody, **characterised in that**
- the amino acid sequence of the variable region of the light chain (V_{L}) is at least 90 % identical to the amino acid sequence of SEQ ID NO: 1 and the amino acid sequence of the variable region of the heavy chain (V_{H}) is at least 90 % identical to SEQ ID NO: 3, and
- the antibody binds low density lipoprotein (LDL), in particular LDL cholesterol.

2. The purified antibody according to Claim 1, **characterised in that** the amino acid sequence of the variable region of the light chain (V_{L}) is at least 95 % identical to SEQ ID NO: 1 and the amino acid sequence of the variable region of the heavy chain (V_{H}) is at least 95 % identical to SEQ ID NO: 3.

3. The purified antibody according to Claim 1 or 2, **characterised in that** the antibody binds more strongly to low density lipoproteins (LDLs) and to very low density lipoproteins (VLDLs) than to high density lipoproteins (HDLs).

4. The antibody according to one of the preceding claims, **characterised in that** the antibody and the low density lipoproteins (LDLs) found in the human and animal body have complementary carbohydrate structures.

5. The antibody according to Claim 1, **characterised in that** the nucleic acid sequence of the variable region of the light chain (V_{L}) is at least 90 % identical to SEQ ID NO: 2 and the nucleic acid sequence of the variable region of the heavy chain (V_{H}) is at least 90 % identical to SEQ ID NO: 4.

6. The antibody according to one of Claims 1 or 2, **characterised in that** the antibody is coded by nucleic acid sequences, which are identical to the nucleotides 67-99 (CDR1), 145-165 (CDR2) and 262-288 (CDR3) of SEQ ID NO: 2 and are identical to the nucleotides 91-105 (CDR1), 148-198 (CDR2) and 295-330 (CDR3) of SEQ ID NO: 4.

7. An antibody, containing the complementarity-determining regions (CDRs), which, in the heavy chain (V_{H}), have the amino acid sequences Ser-Tyr-Ala-Met-His (CDR1), Val-Ile-Ser-Tyr-Asp-Gly-Ser-Asn-Lys-Tyr-Tyr-Ala-Asp-Ser-Val-Lys-Gly (CDR2) and Asp-Arg-Leu-Ala-Val-Ala-Gly-Lys-Thr-Phe-Asp-Tyr (CDR3) of SEQ ID NO: 3, and
which, in the light chain, have the amino acid sequences (V_{L}) Ser-Gly-Asp-Lys-Leu-Gly-Asp-Lys-Tyr-Ala-Cys (CDR1), Gln-Asp-Ser-Lys-Arg-Pro-Ser (CDR2) and Gln-Ala-Trp-Asp-Ser-Ser-Ile-Val-Val (CDR3) of SEQ ID NO: 1, and wherein the antibody binds low density lipoprotein (LDL), in particular LDL cholesterol.

8. A method for producing a purified antibody according to one of the preceding claims by means of hybridoma technology, **characterised in that** the hybridoma cells are obtained by fusion of heteromyeloma cells HAB-1 and their sub-clones with B-lymphocytes removed from human milk, lymph nodes or blood.

9. The purified antibody according to one of Claims 1 to 7, **characterised in that** the antibody can be produced by the method claimed in Claim 8.

10. The antibody according to one of Claims 1 to 9, **characterised in that** the antibody is a monoclonal antibody.

11. The antibody according to Claim 10, **characterised in that** the antibody is a human monoclonal antibody.

12. The antibody according to one of Claims 1 to 11 in combination with conventional additives and/or carrier substances for use as a drug having a fat-reducing effect.

13. The antibody according to one of Claims 1 to 11 for use as a drug for reducing low density lipoprotein (LDL) in the blood.

14. The antibody according to one of Claims 1 to 11 for use as a drug for reducing LDL cholesterol.

15. The antibody according to one of Claims 1 to 11 for use as a drug for the treatment of renal diseases.

16. The antibody for use according to Claim 15 for the production of a drug for the treatment of glomerulonecrosis.

## Revendications

1. Anticorps purifié, **caractérisé en ce que**
- la séquence en acides aminés de la région variable de la chaîne légère (V_{L}) est identique à au moins 90 % à la SEQ ID NO : 1 et la séquence d'acide nucléique de la région variable de la chaîne lourde (V_{H}) est identique à au moins 90 % à la SEQ ID NO : 3 et
- l'anticorps lie la lipoprotéine de basse densité (LDL), en particulier le cholestérol LDL.

2. Anticorps purifié selon la revendication 1, **caractérisée en ce que** la séquence des acides aminés de la région variable de la chaîne légère (V_{L}) est identique à moins 95 % à la SEQ ID NO : 1 et la séquence des acides aminés de la région variable de la chaîne lourde (V_{H}) est identique à au moins 95 % à la SEQ ID NO : 3.

3. Anticorps purifié selon revendication 1 ou 2, **caractérisé en ce que** la liaison de l'anticorps à des lipoprotéines de basse densité (LDL) et à des lipoprotéines de très basse densité (VLDL) est plus forte que la liaison à des lipoprotéines de haute densité (HDL).

4. Anticorps selon l'une des revendications précédentes, **caractérisé en ce que** ledit anticorps et les lipoprotéines de basse densité (LDL), présentes dans le corps humain et le corps animal sont dotées de structures d'hydrate de carbone complémentaires.

5. Anticorps selon la revendication 1, **caractérisé en ce que** la séquence d'acide nucléique de la région variable de la chaîne légère (V_{L}) est identique à au moins 90 % à la SEQ ID NO : 1 et la séquence d'acide nucléique de la région variable de la chaîne lourde (V_{H}) est identique à au moins 90 % à la SEQ ID NO : 4.

6. Anticorps selon l'une des revendications 1 à 5, **caractérisé en ce que** ledit anticorps est codé par des séquence d'acide nucléique, qui sont identiques aux nucléotides 67 - 99 (CDR1), 145 - 165 (CDR2) et 262 - 288 (CDR3) de la SEQ ID NO : 2 et sont identiques aux nucléotides 91 - 105 (CDR1), 148 - 198 (CDR2) et 295 - 330 (CDR3) de la SEQ ID NO : 4.

7. Anticorps comprenant les régions de détermination de complémentarité (CDR), qui, dans la chaîne lourde (V_{H}), présentent les séquences des acides aminés Ser-Tyr-Ala-Met-His (CDR1), Val-Ile-Ser-Tyr-Asp-Gly- Ser-Asn-Lys-Tyr-Tyr-Ala-Asp-Ser-Val-Lys-Gly (CDR2) et Asp-Arg-Leu-Ala-Val-Ala-Gly-Lys-Thr-Phe-Asp-Tyr (CDR3) de SEQ ID NO : 3, et
qui, dans la chaîne légère (V_{L}), lient les séquences des acides aminés Ser-Gly-Asp-Lys-Leu-Gly-Asp-Lys-Tyr-Ala-Cys (CDR1), Gln-Asp-Ser-Lys-Arg-Pro-Ser (CDR2) et Gln-Ala-Trp-Asp-Ser-Ser.Ile-Val-Val (CDR3) de SEQ ID NO : 1 et sachant que l'anticorps lie la lipoprotéine de basse densité (LDL), en particulier le cholestérol LDL.

8. Procédé de production, par la technique des hybridomes, d'un anticorps purifié selon l'une des revendications précédentes, **caractérisé en ce que** les hybridomes sont obtenus par fusion de cellules d'hétéromyélome HAB-1 et de leurs sous-clones avec des lymphocytes B obtenus à partir de la rate, de ganglions lymphatiques ou de sang humains.

9. Anticorps purifié selon l'une des revendications 1 à 7, **caractérisé en ce que** ledit anticorps est obtenu selon le procédé, objet de la revendication 8.

10. Anticorps purifié selon l'une des revendications 1 à 9, **caractérisé en ce que** ledit anticorps est un anticorps monoclonal

11. Anticorps selon la revendication 10, **caractérisé en ce que** ledit anticorps est un anticorps monoclonal, humain.

12. Anticorps selon l'une des revendications 1 à 11, destiné, combiné avec les excipients et / ou les supports habituels, à être utilisé comme produit pharmaceutique, réducteur de graisse.

13. Anticorps selon l'une des revendications 1 à 11, destiné à être utilisé comme produit pharmaceutique pour faire baisser le taux de lipoprotéine de basse densité dans le sang.

14. Anticorps selon l'une des revendications 1 à 11, destiné à être utilisé comme produit pharmaceutique pour faire baisser le cholestérol LDL.

15. Anticorps selon l'une des revendications 1 à 11, destiné à être utilisé comme produit pharmaceutique pour le traitement des maladies rénales.

16. Anticorps, utilisé selon la revendication 15, pour la fabrication d'un produit pharmaceutique, destiné au traitement de la glomérulonécrose.
